(19) Europäisches Patentamt
European Patent Office
Office européen des brevets

(11) Publication number: **0 095 827**
**B1**

(12) **EUROPEAN PATENT SPECIFICATION**

(45) Date of publication of patent specification: **24.07.85**

(51) Int. Cl.$^4$: **C 07 C 93/06,** A 61 K 31/13

(21) Application number: **83301653.8**

(22) Date of filing: **24.03.83**

(54) **N-substituted derivatives of ethanol amine.**

(30) Priority: **08.04.82 GB 8210447**
**28.04.82 GB 8212355**

(43) Date of publication of application:
**07.12.83 Bulletin 83/49**

(45) Publication of the grant of the patent:
**24.07.85 Bulletin 85/30**

(84) Designated Contracting States:
**CH DE FR GB IT LI NL**

(56) References cited:
**EP-A-0 040 000**
**EP-A-0 041 760**

(73) Proprietor: **BEECHAM GROUP PLC**
**Beecham House Great West Road**
**Brentford Middlesex TW8 9BD (GB)**

(72) Inventor: **Hindley, Richard Mark**
**19 Cockshot Road**
**Reigate Surrey (GB)**

(74) Representative: **Cresswell, Thomas Anthony *et al***
**European Patent Attorney Beecham Pharmaceuticals Great Burgh Yew Tree Bottom Road**
**Epsom Surrey KT18 5XQ (GB)**

**EP 0 095 827 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to derivatives of ethanolamine which have anti-obesity and anti-hyperglycaemic activity, to processes for their production and to their use in medicine.

It has now been discovered that a class of novel ethanolamine derivatives have anti-hyperglycaemic and/or anti-obesity activity. This activity is coupled with low cardiac-stimulant activity for particular members of the class.

According to the present invention there is provided a compound of formula (I):

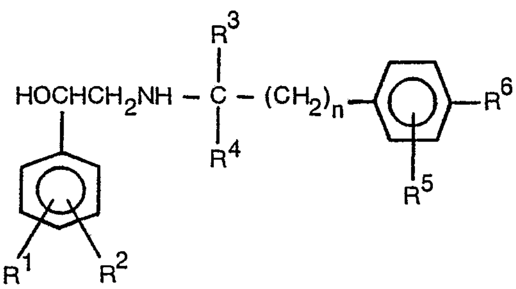

(I)

or a salt thereof,
wherein $R^1$ is hydrogen, halogen or trifluoromethyl,
$R^2$ is hydrogen or halogen,
$R^3$ is hydrogen or methyl,
$R^4$ is hydrogen or methyl,
$R^5$ is hydrogen or halogen,

$R^6$ is 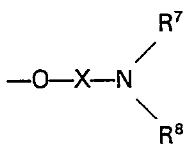

X is $C_{3-20}$ straight or branched alkylene having at least two carbon atoms interposed between the oxygen and nitrogen atoms and having a hydroxyl substituent on a carbon atom not directly bonded to the oxygen or nitrogen atoms
$R^7$ is hydrogen, $C_{1-6}$ straight or branched alkyl, or optionally substituted benzyl,
$R^8$ is hydrogen, $C_{1-6}$ straight or branched alkyl or optionally substituted benzyl,
or

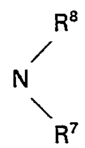

is a 5, 6 or 7 membered saturated heterocyclic group containing a total of one or two heteroatoms, the second hetero-atom, when present, being selected from nitrogen, oxygen and sulphur,
and n is 1 or 2.

It will be appreciated that X cannot represent substituted methylene since this would result in there being only a single carbon atom interposed between the oxygen and nitrogen. Moreover, if X is trimethylene, the hydroxyl substituent must be located on the central carbon atom as this is the only one not directly bonded to the oxygen or nitrogen atoms.

The salts of compounds of formula (I) are preferably, but not essentially, pharmaceutically acceptable, as they are also useful in the preparation of other compounds of formula (I) and in the separation of stereoisomers of compounds of formula (I) when the salt ion is also optically active.

Pharmaceutically acceptable salts of compounds of formula (I) include acid addition salts formed with a pharmaceutically acceptable acid such as hydrochloric acid, hydrobromic acid, orthophosphoric acid, sulphuric acid, methane sulphonic acid, toluenesulphonic acid, acetic acid, propionic acid, lactic acid, citric acid, fumaric acid, malic acid, succinic acid, salicylic acid or acetylsalicylic acid.

Preferably $R^1$ is hydrogen, chlorine or trifluoromethyl.

Preferably $R^6$ is a group

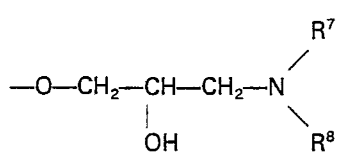

Preferably $R^2$ is hydrogen.

Preferably $R^1$ is located in the meta position with respect to the ethanolamine residue.

Suitable substituents for the benzyl group $R^7$ and/or $R^8$ include halogen, hydroxy, $C_{1-4}$ alkyl, $C_{1-4}$ alkoxy, amino, mono- and di-$(C_{1-4})$alkylamino, nitro, cyano, carboxy and trifluoromethyl substituents.

The compounds of formula (I) all have at least two asymmetric carbon atoms, ie the one bearing the phenyl and hydroxy groups and e.g. the one in the alkylene chain X bearing the hydroxy group. Moreover, when $R^3$ and $R^4$ are different, the carbon atom bearing $R^3$ and $R^4$ is also asymmetric. The compounds may, therefore, exist in at least four and often eight stereoisomeric forms. The present invention encompasses all stereoisomers of the compounds of formula (I) whether free or admixed with other stereoisomers in any proportion and thus includes, for instance, racemic mixtures of enantiomers.

Preferably the carbon atom bearing the phenyl and hydroxy groups has the R-absolute configuration.

When $R^3$ and $R^4$ are different it is preferred that the carbon atom bearing $R^3$ and $R^4$ has the R-absolute configuration.

Most preferably both the carbon atom bearing the phenyl and hydroxy groups and that bearing $R^3$ and $R^4$ have the R-absolute configuration as such compounds are generally the most potent.

The absolute configuration of any compound of formula (I) may be determined by conventional X-ray crystallographic techniques.

The present invention provides a process for producing a compound of formula (I), or a salt thereof, which process comprises reducing an oxo-group and/or double bond and/or cleaving a benzyl group of a compound of formula (II):

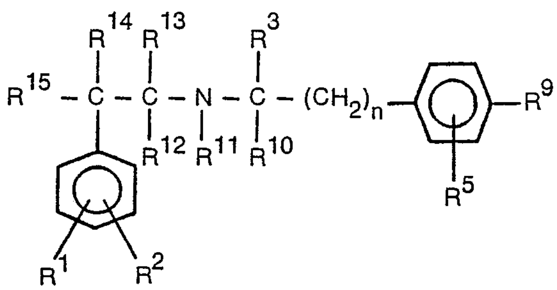

(II)

wherein $R^1$, $R^2$, $R^3$, $R^5$ and n are as defined in relation to formula (I);

$R^9$ is a group $R^6$ as defined in relation to formula (I) or a group reducible to a group $R^6$;

$R^{10}$ is a group $R^4$ as defined in relation to formula (I) or together with $R^{11}$ forms a bond;

$R^{11}$ is hydrogen or benzyl or together with $R^{10}$ or $R^{12}$ forms a bond;

$R^{12}$ is hydrogen or together with $R^{11}$ forms a bond or together with $R^{13}$ forms an oxo-group;

$R^{13}$ is hydrogen or together with $R^{12}$ forms an oxo-group;

$R^{14}$ is hydrogen or together with $R^{15}$ forms an oxo-group;

$R^{13}$ is hydroxyl or together with $R^{14}$ forms an oxo-group;

provided that there is no more than one oxo-group represented by any of $R^{10}$ to $R^{15}$;

and optionally thereafter forming a salt of the compound of formula (I) so formed, and/or converting the compound of formula (I) so formed into a further compound of formula (I).

Where there are two or more reducible moieties in the compound of formula (II) these may be reduced separately in any order or simultaneously.

The aforementioned reductions may be effected by conventional chemical methods or by catalytic methods. Suitably, chemical reduction may be effected with lithium aluminium hydride, sodium cyanoborohydride, sodium borohydride or borane methyl sulphide. Catalytic hydrogenation may be carried out using catalysts such as palladium on charcoal, or platinum, for instance, as platinum oxide.

Reduction by sodium borohydride is conveniently effected in a lower alkanolic solvent such as methanol or ethanol. The reaction is generally carried out at from 0—20°C.

Reduction by lithium aluminium hydride is conveniently effected in a dry, ether solvent such as diethyl ether or tetrahydrofuran at ambient or elevated temperatures.

Catalytic reduction is conveniently effected in a conventional hydrogenation solvent such as a lower alkanol, for instance ethanol. The hydrogenation is generally carried out under hydrogen gas at about 1 to 10 atmospheres pressure and at ambient or elevated temperatures.

Reduction of a compound of formula (II) wherein $R^{11}$ is benzyl is conveniently effected by catalytic hydrogenation, preferably using palladium on charcoal as catalyst.

Preferred aspects of the process comprise reducing a compound of formula (IIA):

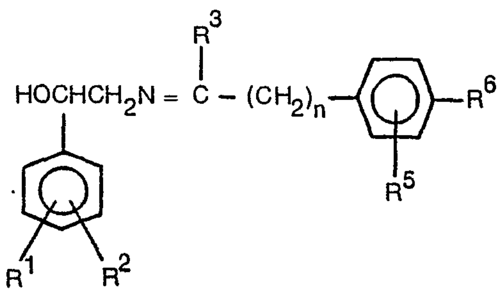

(IIA)

or reducing a compound of formula (IIB):

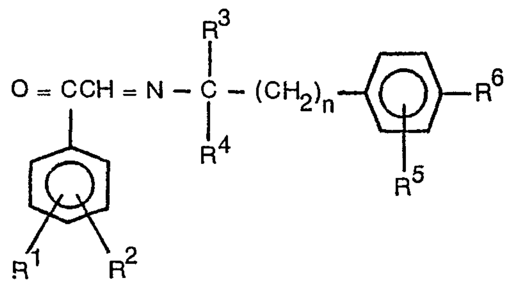

(IIB)

or reducing a compound of formula (IIC):

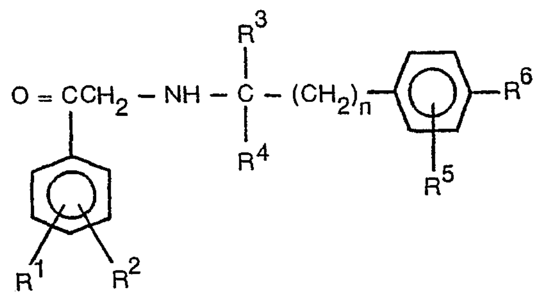

(IIC)

or reducing a compound of formula (IID):

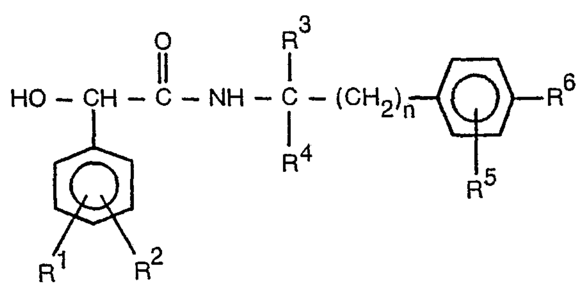

(IID)

or reducing a compound of formula (IIE):

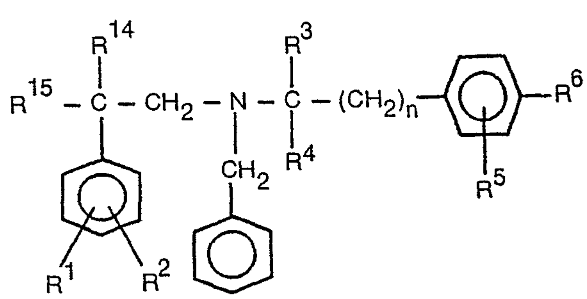

(IIE)

or reducing a compound of formula (IIF):

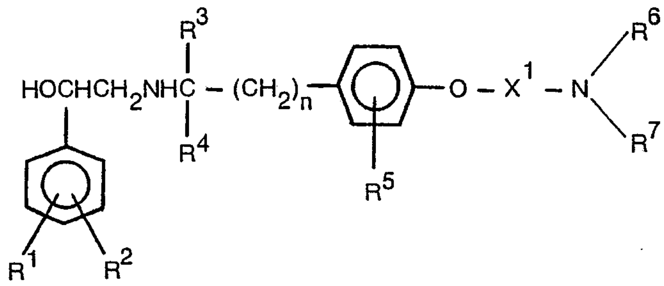

(IIF)

wherein $R^1$, $R^2$, $R^3$, $R^4$, $R^5$, $R^6$ and n are as defined in relation to formula (I) and $R^{14}$ and $R^{15}$ are as defined in relation to formula (II), and $X^1$ is a group corresponding to X as defined in relation to formula (I) but having an oxo substituent in place of the hydroxy substituent.

The present invention also provides another process for producing a compound of formula (I) or a salt thereof, which process comprises reacting a compound of formula (III):

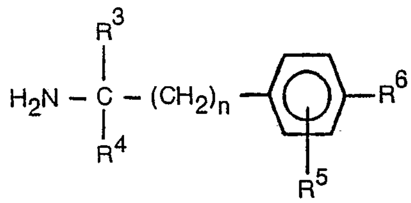

(III)

wherein $R^3$, $R^4$, $R^5$, $R^6$ and n are as defined in relation to formula (I); with a compound of formula (IV):

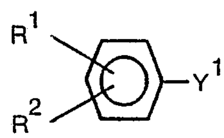

(IV)

wherein $R^1$ and $R^2$ are as defined in relation to formula (I), and $Y^1$ is a group capable of reacting with the amine of formula (III) thus forming a compound of formula (I), and optionally thereafter forming a salt of the compound of formula (I) so formed and/or converting the compound of formula (I) so formed into a further compound of formula (I).

Typical examples of compounds of formula (IV) are compounds of formulae (IVA) and (IVB):

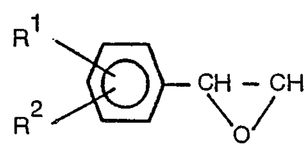

(IVA)

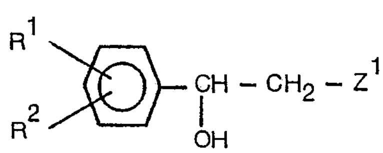

(IVB)

wherein $R^1$ and $R^2$ are as defined in relation to formula (I) and $Z^1$ is a leaving group, preferably halogen or tosyloxy.

The reaction of a compound of formula (III) with a compound of formula (IVA) is conveniently effected in a solvent such as a lower alkanol, preferably ethanol.

The reaction of a compound of formula (III) with a compound of formula (IVB) is conveniently conducted in a solvent, such as dimethyl sulphoxide, at elevated temperature, preferably about 50°C, for about 3 days.

The present invention provides a further process for the production of compounds of formula (I) or salts thereof, which process comprises reacting a compound of formula (V):

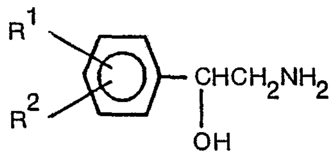

(V)

wherein $R^1$ and $R^2$ are as defined in relation to formula (I); with a compound of formula (VI):

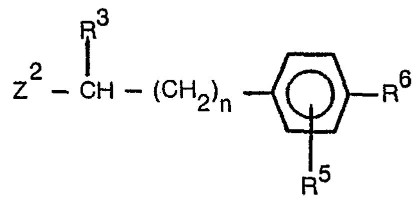

(VI)

wherein $R^3$, $R^5$, $R^6$ and n are as defined in relation to formula (I) and $Z^2$ is a leaving group preferably halogen or tosyloxy.

The reaction of a compound of formula (V) with a compound of formula (VI) is conveniently effected in a solvent such as dimethylsulphoxide at an elevated temperature, preferably about 50°C for about two or three days.

A preferred process for producing compounds of formula (I) comprises the reduction of a compound of formula (IIA), especially using sodium borohydride in methanol at ambient temperatures.

The salts of compounds of formula (I) may be produced by treating the compound of formula (I) with the appropriate acid.

Compounds of formula (I) and salts thereof, produced by the above processes, may be recovered by conventional methods.

Compounds of formula (II) may be produced by reacting a compound of formula (III) as hereinbefore defined with a compound of formula (VII):

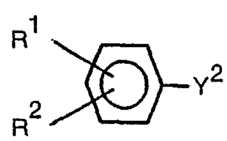

(VII)

wherein $R^1$ and $R^2$ are as defined in relation to formula (I) and $Y^2$ is a group which is capable of reacting with the amine of formula (III) thus forming a compound of formula (II).

Typical examples of compounds of formula (VII) are:

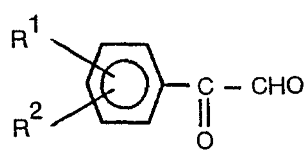

(VIIA)

or its hydrate or hemi-acetal of a lower alkanol;

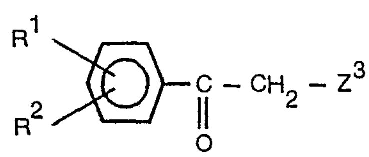

(VIIB)

wherein $Z^3$ is a leaving group, preferably bromine;

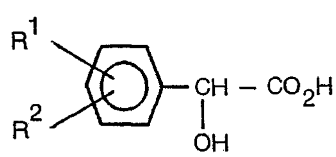

(VIIC)

wherein $R^1$ and $R^2$ are as defined in relation to formula (I).

Conventional conditions compatible with the particular compound of formula (VII) may be used for this reaction. Thus, the reaction of a compound of formula (VIIA) with a compound of formula (III) is conveniently conducted at elevated temperatures under conditions resulting in the removal of the water formed during the reaction. A particularly suitable method is to perform the reaction in a solvent, such as benzene, under reflux and to remove the water azeotropically using a Dean and Stark trap.

The reaction of a compound of formula (VIIB) with a compound of formula (III) is conveniently conducted in a polar organic solvent such as acetonitrile or butanone, at an elevated temperature, for instance under reflux.

The reaction of a compound of formula (VIIC) with a compound of formula (III) is conveniently conducted under standard peptide formation reaction conditions.

Alternatively a compound of formula (II) may be prepared by reacting a compound of formula (VIII):

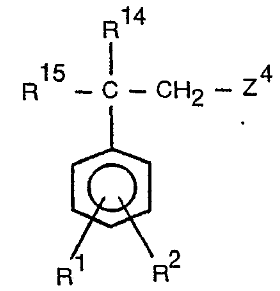

(VIII)

wherein $R^1$, $R^2$, $R^{14}$ and $R^{15}$ are as defined in relation to formula (II);
and $Z^4$ is a leaving group, preferably halogen or tosyloxy;
with a compound of formula (IX):

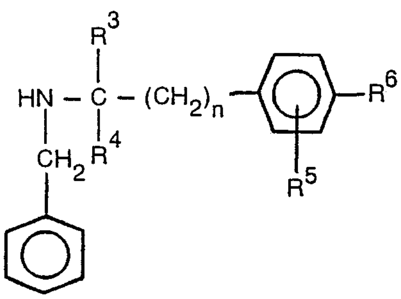

(IX)

wherein $R^3$, $R^4$, $R^5$, $R^6$ and n are as defined in relation to formula (II).

A particularly preferred process for producing certain compounds of formula (II) is reacting a compound of formula (V) as hereinbefore defined with a compound of the formula (X):

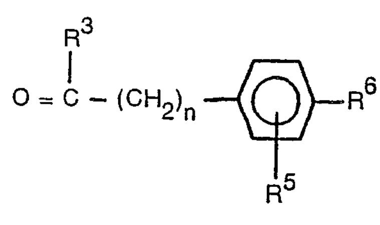

(X)

wherein $R^3$, $R^5$, $R^6$ and n are as defined in relation to formula (I).

The reaction of a compound of formula (V) with a compound of formula (X) is conveniently effected under conditions which result in the removal of water formed during the reaction. A particularly suitable method is to perform the reaction in a solvent, such as benzene, under reflux and to remove the water azeotropically using a Dean and Stark trap.

It is often convenient to prepare the compound of formula (II) and reduce it, in situ, to the desired compound of formula (I) without isolation of the compound of formula (II).

Those compounds of formula (I) having more than one asymmetric carbon atom may be separated into diastereoisomeric pairs of enantiomers by, for example, fractional crystallisation from a suitable solvent such as ethyl acetate. The pair of enantiomers thus obtained may be separated into individual stereoisomers by conventional means such as by the use of an optically active acid as a resolving agent.

Suitable optically active acids which may be used as resolving agents are described in "Topics in Stereochemistry", Vol. 6, Wiley Interscience, 1971, Allinger, N.L., and Eliel, W.L. Eds.

Alternatively any enantiomer of a compound of formula (I) may be obtained by stereospecific synthesis using optically pure starting materials of known configuration.

By using single enantiomers of a compound of formula (III) and of a compound of formula (VIIC) a stereospecific synthesis of a compound of formula (II) is achieved. This may then be reduced to a compound of formula (I) without altering the configuration of the asymmetric carbon atoms. Thus, for example, reaction of a compound of formula (VIIC) with the *R*-absolute configuration and a single enantiomer of a compound of formula (III), in which the carbon atom bearing $R^3$ and $R^4$ has the *R*-absolute configuration and the propanol residue has either the *R*- or *S*-absolute configuration, would afford a compound of formula (II) and, on reduction, a single enantiomer of the compound of formula (I) with the *R,R,R*- or *R,R,S*-absolute configuration.

By reacting a single enantiomer of a compound of formula (III) with a single enantiomer of a compound of formula (IVA) or (IVB), the direct stereospecific synthesis of a single enantiomer of a compound of formula (I) is effected. Thus, for example, reaction of a compound of formula (IVA) with the *R*-absolute configuration with a single enantiomer of a compound of formula (III) in which the carbon atom bearing $R^3$ and $R^4$ has the *R*-absolute configuration and the propanol residue has the *R* or *S*-absolute configuration would afford a compound of formula (I) with the *R,R,R*- or *R,R,S*-absolute configuration.

A compound of formula (I) or a pharmaceutically acceptable salt thereof (hereinafter "the drug") may be administered as the pure drug, however, it is preferred that the drug be administered as a pharmaceutical composition also comprising a pharmaceutically acceptable carrier.

Accordingly, the present invention also provides a pharmaceutical composition comprising a compound of formula (I) or a pharmaceutically acceptable salt thereof with a pharmaceutically acceptable carrier therefor.

As used herein the terms "pharmaceutical composition" and "pharmaceutically acceptable" embrace compositions and ingredients for both human and veterinary use.

Usually the compositions of the present invention will be adapted for oral administration although compositions for administration by other routes, such as by injection are also envisaged.

Particularly suitable compositions for oral administration are unit dosage forms such as tablets and capsules. Other fixed unit dosage forms, such as powders presented in sachets, may also be used.

In accordance with conventional pharmaceutical practice the carrier may comprise a diluent, filler, disintegrant, wetting agent, lubricant, colourant, flavourant or the like.

Typically carriers may, therefore, comprise such agents as microcrystalline cellulose, starch, sodium starch glycollate, polyvinylpyrrolidone, polyvinylpolypyrrolidone, magnesium stearate, sodium lauryl sulphate, sucrose and the like.

Most suitably the composition will be provided in unit dose form. Such unit doses will normally comprise 0.1 to 500 mg of the drug, more usually 0.1 to 250 mg and favourably 0.1 to 100 mg.

The present invention further provides the compounds of formula I for a method for treating obesity in humans or domestic mammals, which method comprises administering an effective, non-toxic amount of a compound of formula (I) or a pharmaceutically acceptable salt thereof to obese humans or domestic mammals.

The present invention further provides the compounds of formula I for a method for treating hyperglycaemia in humans which method comprises administering an effective, non-toxic amount of a compound of formula (I) or a pharmaceutically acceptable salt thereof to hyperglycaemic humans.

Conveniently, the drug may be administered as a pharmaceutical composition as hereinbefore defined, and this forms a particular aspect of the present invention.

In treating hyperglycaemic or obese humans the drug may be taken in doses, such as those described above, one to six times a day in a manner such that the total daily dose for a 70 kg adult will generally be about 0.1 to 1000 mg, and more usually about 1 to 500 mg.

In treating hyperglycaemic or obese animals, especially dogs, the drug may be administered by mouth, usually once or twice a day and at about 0.025 mg/kg to 10 mg/kg, for examples 0.1 mg/kg to 2 mg/kg.

The invention will now be illustrated with reference to the following Examples, which are not intended to limit the scope in any way.

Example 1

N-[2-(4-{1-(2-Hydroxy-3-isopropylaminopropyloxy)}phenyl)-1-methylethyl]-2-hydroxy-2-(3-trifluoromethylphenyl)ethanamine

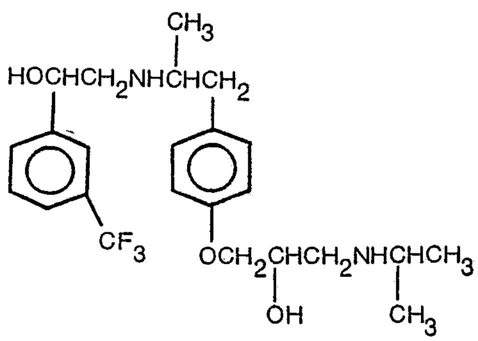

A mixture of 1-[4-{1-(2-hydroxy-3-isopropylaminopropyloxy)}phenyl]propan-2-one (2.0 g) and 2-hydroxy-2-(3-trifluoromethylphenyl)ethanamine (1.55 g) in benzene (100 ml) was heated under reflux using a Dean and Stark head until the theoretical amount of water had been collected. The solution was cooled, the solvent evaporated, the residue dissolved in methanol (50 ml) and hydrogenated in the presence of platinum (50 mg, platinum oxide) until hydrogen uptake ceased. Filtration through diatomaceous earth followed by evaporation to dryness gave an oil which was purified by chromatography on silica gel in 10% methanoldichloromethane. Further purification by chromatography on silica gel in methanol (20%), dichloromethane (79.5%) and ammonia solution (0.880; 0.5%) followed by treatment with methanolic hydrogen chloride gave N-[2-(4-{1-(2-Hydroxy-3-isopropylaminopropyloxy)}phenyl)-1-methylethyl]-2-hydroxy-2-(3-trifluoromethylphenyl)ethanamine dihydrochloride monohydride, mp 110—118°C.

*$^1H$ nmr*

τ($DMSOd_6$ + $D_2O$) 8.70–8.90 (9H, complex), 5.7—7.5 (10H, complex), 4.7—5.2 (2H, complex), 3.1 (2H, d), 2.8 (2H, d), 2.25 (4H, complex).

Example 2

N-[2-(4-{1-(2-hydroxy-3-dimethylaminopropyloxy)}phenyl)-1-methylethyl]-2-hydroxy-2-(3-chlorophenyl)ethanamine

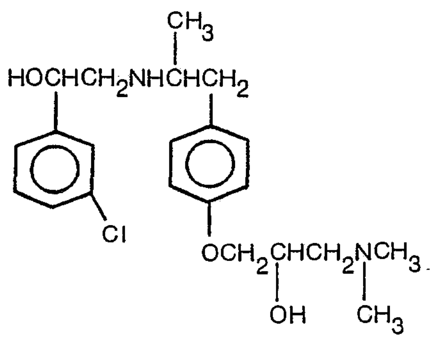

1-[4-{1-(2-Hydroxy-3-dimethylaminopropyloxy)}phenyl]propan-2-one (1.85 g) and 2-hydroxy-2-(3-chlorophenyl)ethanamine (1.10 g) were condensed by the method described in Example 1. The residue, after removal of the benzene, was dissolved in methanol (40 ml), cooled in an ice bath and treated portionwise with sodium borohydride (2 g). After stirring at ambient temperature for 2 hours, water (100 ml) was added. The solution was extracted with dichloromethane and the organic extracts dried (magnesium sulphate), filtered and evaporated to dryness. Chromatography of the residue on silica gel in methanol (20%), dichloromethane (79.5%) and ammonia solution (0.880; 0.5%) followed by treatment with methanolic hydrogen chloride, gave N-[2-(4-{1-(2-hydroxy-3-dimethylaminopropyloxy)}phenyl)-1-methylethyl]-2-hydroxy-2-(3-chlorophenyl)ethanamine dihydrochloride monohydrate, mp 110—115°C.

*$^1H$ nmr*

τ($DMSOd_6$ + $D_2O$) 8.9 (3H, d), 5.8—7.5 (15H, complex), 5.6—5.9 (1H, multiplet), 4.8—5.1 (1H, multiplet), 3.1 (2H, d), 2.8 (2H, d), 2.5 (4H, complex).

Example 3

N-[2-(4-{1-(2-Hydroxy-3-isopropylaminopropyloxy)}phenyl)1-methylethyl]-2-hydroxy-2-(3-chlorophenyl)ethanamine

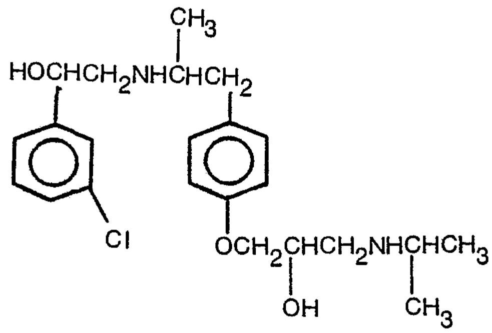

1-[4-{1-(2-Hydroxy-3-isopropylaminopropoxy)}phenyl]propan-2-one (3.4 g) and 2-hydroxy-2-(3-chlorophenyl)ethanamine (2.3 g) were condensed and subsequently reduced with platinum and hydrogen and purified by the method described in Example 1. Treatment of the product with methanolic hydrogen chloride, evaporation and trituration of the resultant gum with ethyl acetate gave N-[2-(4-{1-(2-hydroxy-3-isopropylaminopropyloxy)}phenyl)1-methylethyl]-2-hydroxy-2-(3-chlorophenyl)ethanamine dihydrochloride sesquihydrate, mp 76—82°C.

*$^1$H nmr*

τ(DMSO$d_6$ + $D_2O$) 8.7—8.9 (9H, complex), 5.5—7.3 (11H, complex), 4.9 (1H, multiplet), 3.1 (2H, d), 2.8 (2H, d) 2.55 (4H, complex).

Description 1

1-[4-{1-(2,3-Dihydroxypropyloxy)}phenyl]propan-2-one ethylene ketal

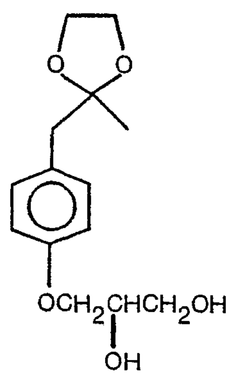

To a solution of potassium hydroxide (5.0 g) in water (150 ml) containing potassium iodide (5.6 g) was added 4-hydroxyphenylpropanone ethylene ketal (9.6 g) and the mixture was stirred at ambient temperature for 30 min. 1-Chloro-2,3-dihydroxypropane (6.1 g) was added and the mixture was boiled under reflux with stirring for 6 hours. After cooling the mixture was extracted with dichloromethane (2 × 100 ml), the organic extracts were washed with saturated brine (1 × 100 ml), dried (magnesium sulphate) filtered and evaporated to dryness to give the title compound (7.6 g) as an oil which was used without further purification.

*$^1$H nmr*

τ($CDCl_3$) 8.7 (3H, s), 7.15 (2H, s), 5.6—6.5 (11H, complex, 2H exchange with $D_2O$), 3.15 (2H, d), 2.80 (2H,d).

Description 2

1-[4-{1-(2-Hydroxy-3-{4-toluenesulphonyloxy}propyloxy}phenyl]propan-2-one ethylene ketal

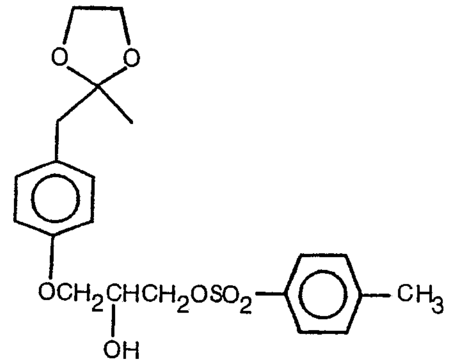

To a solution of 1-[4-{1-(2,3-dihydroxyproploxy)}phenyl]propan-2-one ethylene ketal (7.5 g) in pyridine (50 ml) at 0°C was added 4-toluenesulphonyl chloride (5.8 g) in portions with stirring. The mixture was then stirred at 0—5°C for 1 hour. After dilution with water (100 ml) and neutralisation with hydrochloric acid (10%) the mixture was extracted with diethyl ether, the organic phase washed with saturated sodium bicarbonate, dried (magnesium sulphate), filtered and evaporated to dryness to give the title compound (8.5 g) as an oil.

$^1$*H nmr*

τ($CDCl_3$) 8.7 (3H, s), 7.6 (3H, s), 7.15 (2H, s), 3.05 (1H, s, exchanges with $D_2O$), 5.7—6.35 (9H, complex), 3.25 (2H, d), 2.5—2.9 (4H, 2 superimposed doublets), 2.2 (2H, d).

Description 3

1-[4-{1-(2-Hydroxy-3-isopropylaminopropyloxy)}phenyl]propan-2-one ethylene ketal

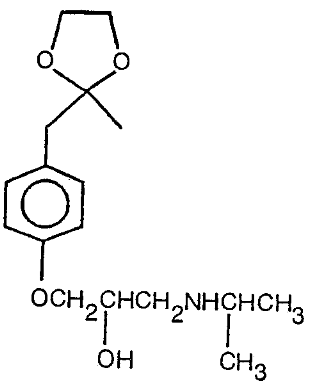

Isopropylamine (10 ml) was added to a solution of 1-[4-{1-(2-hydroxy-3-{4-toluenesulphonyloxy}-propyloxy)}phenyl]propan-2-one ethylene ketal (8.6 g) in dimethylsulphoxide (50 ml) and the mixture was stirred at ambient temperature for 18 hours. The solution was added to water (250 ml) extracted with diethyl ether (2 × 250 ml) and, after washing with brine, the organic phase was dried (magnesium sulphate), filtered and evaporated to dryness to give the title compound (4.3 g) as an oil.

$^1$*H nmr*

τ($CDCl_3$) 8.9 (6H, d), 8.7 (3H, s), 7.0—7.4 (7H, complex, 2H exchange with $D_2O$), 5.9—6.4 (7H, complex), 3.2 (2H, d), 2.8 (2H, d).

Description 4

1-[4-{1-(2-Hydroxy-3-isopropylaminopropyloxy)}phenyl]propan-2-one

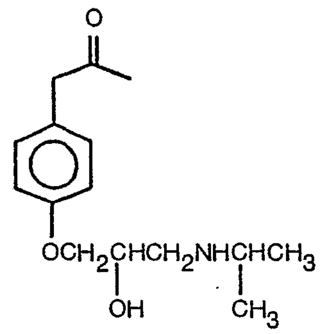

To a solution of 1-[4-{1-(2-hydroxy-3-isopropylamino propyloxy)}phenyl]propan-2-one ethylene ketal (4.2 g) in methanol (50 ml) was added hydrochloric acid (10%, 50 ml) and the solution was allowed to stand at ambient temperature for 5 hours. The solution was basified with saturated sodium bicarbonate solution, extracted with diethyl ether, the organic extracts dried (magnesium sulphate), filtered and evaporated to give the title compound (3.44 g) as an oil.

*$^{1}H$ nmr*

τ($CDCl_3$) 8.9 (6H, d), 7.9 (3H, s), 7.2 (5H, complex, 2H exchange with $D_2O$, 5.8—6.5 (5H, complex), 3.2 (2H, d), 2.9 (2H, d).

Description 5

1-[4-{1-(2-Hydroxy-3-dimethylaminopropyloxy)}phenyl]propan-2-one

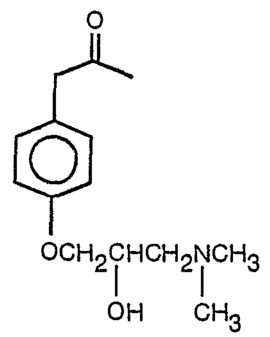

Methylamine was passed through a solution of 1-[4-{1-(2-hydroxy-3-{4-toluenesulfonyloxy}-propyloxy)}phenyl]propan-2-one ethylene ketal (5.0 g) in dimethylsulphoxide (50 ml) for 2 hours. The mixture was allowed to stand at ambient temperature overnight. Water was added, the solution extracted with ether, and the organic layer dried (magnesium sulphate) and evaporated to give 1-[4-{1-(2-hydroxy-3-dimethylaminopropyloxy)}phenyl]propan-2-one ethylene ketal (3.72 g), from which the title compound (1.85 g) was obtained as in Description 4.

*$^{1}H$ nmr*

τ($CDCl_3$) 7.9 (3H, s), 7.7 (6H, s), 7.5 (2H, d), 6.4 (2H, s), 6.0 (3H, complex), 5.5 (1H, s, exchanges with $D_2O$), 3.2 (2H, d), 2.9 (2H, d).

Demonstration of Effectiveness of Compounds

(i) *Effect on energy expenditure*

The effect of the compounds on the energy expenditure of mice was demonstrated by means of the following procedure:

Female CFLP mice each weighing approximately 24 g, were given food and water *ad lib* before and during the experiment. The compounds were dissolved in water by addition of one mole of hydrochloric acid per mole of compound and these solutions were administered orally to each of 12 mice. A further 12 mice were dosed orally with water. The mice were placed in boxes through which the air was drawn and the oxygen content of the air leaving the boxes was measured. The energy expenditure of the mice was calculated for 21 hours after dosing from the volume of air leaving the boxes and its oxygen content, following the principles described by J.B. de V. Weir, *J. Physiol.* (London) *109,* 1—9 (1949). The food intake of the mice was measured over this same period of 21 hours. The results (Table 1) are expressed as a percentage of the mean food intake or rate of energy expenditure of the mice dosed with water.

| Compound of Example No. | Dose (mg/kg po) | Mean Energy Expenditure % (0–21h) | Mean Food Intake % |
|---|---|---|---|
| 1 | 29.0 | 115 | 94 |
| 2 | 27.7 | 123 | 88 |
| 3 | 28.9 | 124 | 92 |

(ii) *Hypoglycaemic Activity*

Female CFLP mice, each weighing approximately 25 g were fasted for 24 hours prior to the study. The compounds under study were administered orally as an aqueous solution to each of 6 mice. 6 mice were given water as a control. 30 minutes later a blood sample (20 µl) was obtained from the tail for the analysis of blood glucose. Immediately after taking this blood sample, glucose (1 g/kg body weight) was administered subcutaneously to each mouse. Blood samples were then obtained from each mouse at 30 minute intervals for 120 minutes.

Compounds that produced a significant (P 0.05) reduction of blood glucose, compared with control mice given water, at any time interval, were considered active. The area under the blood glucose curve over the 2 hour period after the administration of the glucose was calculated for each compound and compared with the value for control animals.

| Compound of Example No. | Dose µmol/kg p.o. | Reduction in Area % |
|---|---|---|
| 1 | 12.5 | 63 |
| 2 | 2.5 | 57 |
| 3 | 12.5 | 59 |

**Claims**

1. A compound of formula (I):

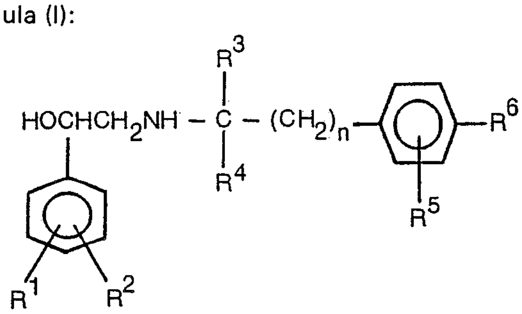

(I)

or a salt thereof,
wherein $R^1$ is hydrogen, halogen or trifluoromethyl,
$R^2$ is hydrogen or halogen,
$R^3$ is hydrogen or methyl,
$R^4$ is hydrogen or methyl,
$R^5$ is hydrogen or halogen,

$R^6$ is 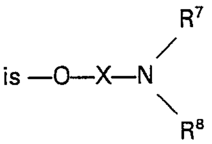

X is $C_{3-20}$ straight or branched alkylene having at least two carbon atoms interposed between the oxygen and nitrogen atoms and having a hydroxyl substituent on a carbon atom not directly bonded to the oxygen or nitrogen atoms

$R^7$ is hydrogen, $C_{1-6}$ straight or branched alkyl, or optionally substituted benzyl,
$R^8$ is hydrogen, $C_{1-6}$ straight or branched alkyl or optionally substituted benzyl,
or

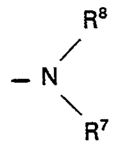

is a 5, 6 or 7 membered saturated heterocyclic group containing a total of one or two heteroatoms, the second hetero-atom, when present, being selected from nitrogen, oxygen and sulphur,
and n is 1 or 2.

2. A compound according to claim 1 wherein $R^1$ is hydrogen, chlorine or trifluoromethyl.

3. A compound according to claim 1 wherein $R^6$ is a group

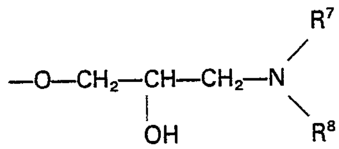

4. A compound according to claim 1 wherein $R^2$ is hydrogen.

5. A process for producing a compound of formula (I) as defined in claim 1, or a salt thereof, which process comprises reducing an oxo-group and/or double bond and/or cleaving a benzyl group of a compound of formula (II):

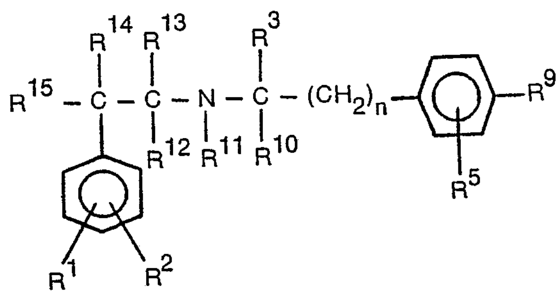

(II)

wherein $R^1$, $R^2$, $R^3$, $R^5$ and n are as defined in relation to formula (I);
$R^9$ is a group $R^6$ as defined in relation to formula (I) or a group reducible to a group $R^6$;
$R^{10}$ is a group $R^4$ as defined in relation to formula (I) or together with $R^{11}$ forms a bond;
$R^{11}$ is hydrogen or benzyl or together with $R^{10}$ or $R^{12}$ forms a bond;
$R^{12}$ is hydrogen or together with $R^{11}$ forms a bond or together with $R^{13}$ forms an oxo-group;
$R^{13}$ is hydrogen or together with $R^{12}$ forms an oxo-group;
$R^{14}$ is hydrogen or together with $R^{15}$ forms an oxo-group;
$R^{15}$ is hydroxyl or together with $R^{14}$ forms an oxo-group;
provided that there is no more than one oxo-group represented by any of $R^{10}$ to $R^{15}$;
and optionally thereafter forming a salt of the compound of formula (I) so formed, and/or converting the compound of formula (I) so formed into a further compound of formula (I).

6. A process for producing a compound of formula (I) as defined in claim 1, or a salt thereof, which process comprises reacting a compound of formula (III):

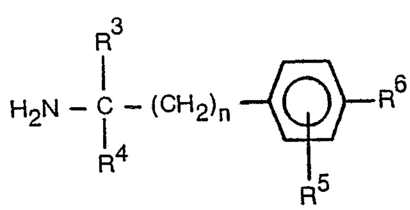

(III)

wherein $R^3$, $R^4$, $R^5$, $R^6$ and n are as defined in relation to formula (I);
with a compound of formula (IV):

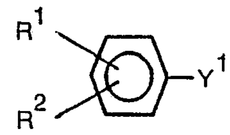

(IV)

wherein $R^1$ and $R^2$ are as defined in relation to formula (I), and $Y^1$ is a group capable of reacting with the amine of formula (III) thus forming a compound of formula (I), and optionally thereafter forming a salt of the compound of formula (I) so formed and/or converting the compound of formula (I) so formed into a further compound of formula (I).

7. A process for producing a compound of formula (I) as defined in claim 1, or salts thereof, which process comprises reacting a compound of formula (V):

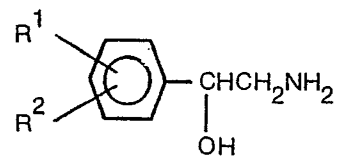

(V)

wherein $R^1$ and $R^2$ are as defined in relation to formula (I);

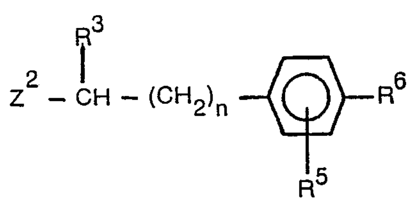

(VI)

wherein $R^3$, $R^5$, $R^6$ and n are as defined in relation to formula (I) and $Z^2$ is a leaving group preferably halogen or tosyloxy.

8. A pharmaceutical composition comprising a compound of formula (I) as defined in claim 1 and a pharmaceutically acceptable carrier therefor.

9. A composition according to claim 8 in unit dose from comprising from 0.1 to 500 mg of the compound of formula (I).

10. A compound of formula (I) for use in treating the human or animal body.

11. A compound according to claim 1 which is selected from:
N-[2-(4-{1-(2-hydroxy-3-isopropylaminopropyloxy)}phenyl)-1-methylethyl]-2-hydroxy-2-(3-trifluoromethylphenyl)ethanamine;
N-[2-(4-{1-(2-hydroxy-3-dimethylaminopropyloxy)}phenyl)-1-methylethyl]-2-hydroxy-2-(3-chlorophenyl)ethanamine;
N-[2-(4-{1-2-hydroxy-3-isopropylaminopropyloxy)}phenyl)1-methylethyl]-2-hydroxy-2-(3-chloroenyl)-ethanamine.

**Patentansprüche**

1. Eine Verbindung der Formel (I):

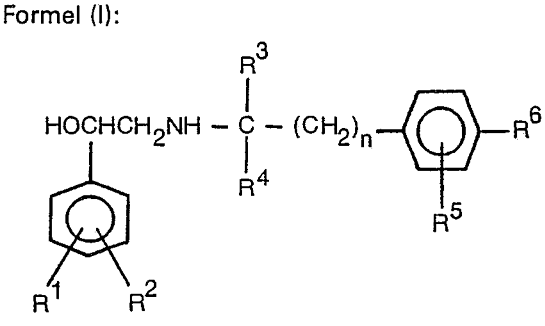

(I)

oder ein Salz derselben,
in welcher

$R^1$ Wasserstoff, Halogen oder Trifluormethyl bedeutet,
$R^2$ Wasserstoff oder Halogen,
$R^3$ Wasserstoff oder Methyl,
$R^4$ Wasserstoff oder Methyl,
$R^5$ Wasserstoff oder Halogen bedeutet,

$R^6$ die folgende Strukturformel hat: 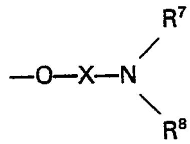

X ein geradkettiges oder verzweigtes $C_{3-20}$-Alkylen mit mindestens 2 Kohlenstoffatomen ist, die zwischen dem Sauerstoff- und dem Stickstoffatom angeordnet sind, und einem Hydroxylsubstituenten an einem Kohlenstoffatom, welches nicht direkt an das Sauerstoff-bzw. Stickstoffatom gebunden ist,

$R^7$ Wasserstoff, ein geradkettiges oder verzweigtes $C_{1-6}$-Alkyl oder eine gegebenenfalls substituiertes Benzyl bedeutet,

$R^8$ Wasserstoff, ein geradkettiges oder verzweigtes $C_{1-6}$-Alkyl oder ein gegebenenfalls substituiertes Benzyl bedeutet, oder

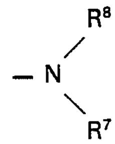

eine 5-, 6- oder 7-gliedrige gesättigte heterozyklische Gruppe mit insgesamt 1 oder 2 Heteroatomen ist, wobei das zweite Heteroatom, falls vorhanden, ausgewählt ist aus Stickstoff, Sauerstoff und Schwefel, und n den Wert 1 oder 2 hat.

2. Eine Verbindung nach Anspruch 1, in welcher $R^1$ Wasserstoff, Chlor oder Trifluormethyl bedeutet.

3. Eine Verbindung nach Anspruch 1, in welcher $R^6$ eine Gruppe der Strukturformel

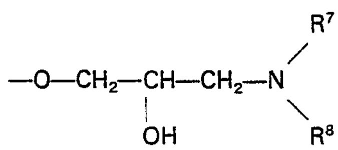

4. Eine Verbindung nach Anspruch 1, in welcher $R^2$ Wasserstoff ist.

5. Ein Verfahren zur Herstellung einer Verbindung der Formel(I) wie in Anspruch 1 beansprucht, oder eines Salzes derselben, welches Verfahren die Reduktion einer Oxo-Gruppe und/oder einer Doppelbindung und/oder die Abspaltung einer Benzylgruppe einer Verbindung der Formel (II):

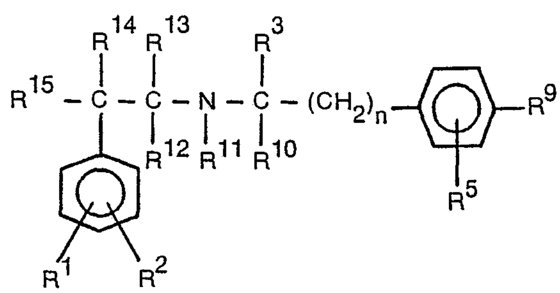

(II)

in welcher $R^1$, $R^2$, $R^3$, $R^5$ und n wie in bezug auf Formel (I) definiert sind;

$R^9$ eine Gruppe $R^6$, wie in bezug auf Formel (I) definiert, oder eine zu einer Gruppe $R^6$ reduzierbare Gruppe ist;

$R^{10}$ eine Gruppe $R^4$, wie in bezug auf Formel (I) definiert, ist oder zusammen mit $R^{11}$ eine Bindung bildet;

$R^{11}$ Wasserstoff oder Benzyl ist oder zusammen mit $R^{10}$ oder $R^{12}$ eine Bindung bildet;

$R^{12}$ Wasserstoff ist oder zusammen mit $R^{11}$ eine Bindung bildet oder zusammen mit $R^{13}$ eine Oxogruppe bildet;

$R^{13}$ Wasserstoff ist oder zusammen mit $R^{12}$ eine Oxogruppe bildet;

$R^{14}$ Wasserstoff ist oder zusammen mit $R^{15}$ eine Oxogruppe bildet;

$R^{15}$ Hydroxyl ist oder zusammen mit $R^{14}$ eine Oxogruppe bildet;

vorausgesetzt, daß nur eine Oxogruppe durch eine der Gruppen $R^{10}$ bis $R^{15}$ dargestellt wird; und gegebenenfalls anschließend die Bildung eines Salzes der so gebildeten Verbindung der Formel (I) und/oder die Umwandlung der so gebildeten Verbindung der Formel (I) in eine weitere Verbindung der Formel (I) umfaßt.

6. Ein Verfahren zur Herstellung einer Verbindung der Formel (I), wie in Anspruch 1 definiert, oder eines Salzes derselben, welches Verfahren die Umsetzung einer Verbindung der Formel (III):

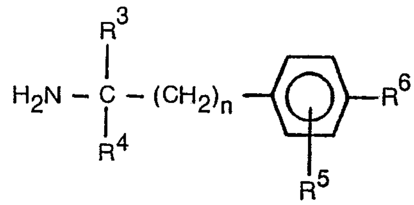

(III)

in welcher $R^3$, $R^4$, $R^5$, $R^6$ und n wie in bezug auf Formel (I) definiert sind; mit einer Verbindung der Formel (IV):

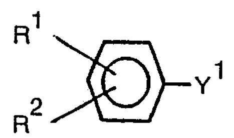

(IV)

in welcher $R^1$ und $R^2$ wie in bezug auf Formel (I) definiert sind und $Y^1$ eine Gruppe ist, welche mit dem Amin der Formel (III) umgesetzt werden kann und somit eine Verbindung der Formel (I) gebildet wird,
und gegebenenfalls anschließend die Bildung eines Salzes der so gebildeten Verbindung der Formel (I) und/oder die Umwandlung der so gebildeten Verbindung der Formel (I) in eine weitere Verbindung der Formel (I) umfaßt.

7. Eine Verfahren zur Herstellung einer Verbindung der Formel (I) wie in Anspruch 1 definiert, oder von Salzen derselben, welches Verfahren die Umsetzung einer Verbindung der Formel (V):

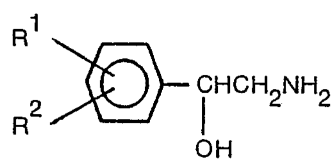

(V)

in welcher $R^1$ und $R^2$ wie in bezug auf Formel (I) definiert sind; mit einer Verbindung der Formel (VI):

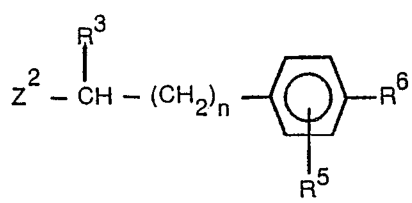

(VI)

in welcher $R^3$, $R^5$, $R^6$ und n wie in bezug auf Formel (I) definiert sind, und $Z^2$ eine freisetzbare Gruppe, vorzugsweise Halogen oder Tosyloxy ist.

8. Eine pharmazeutische Zusammensetzung umfassend eine Verbindung der Formel (I) wie in Anspruch 1 definiert und einen pharmazeutisch verträglichen Träger dafür.

9. Eine Zusammensetzung nach Anspruch 8 in Einzeldosisform umfassend von 0,1 bis 500 mg der Verbindung der Formel (I).

10. Eine Verbindung der Formel (I) zur Verwendung bei der Behandlung von Mensch und Tier.

11. Eine Verbindung gemäß Anspruch 1, die ausgewählt ist aus:
N-[2-(4-{1-(2-Hydroxy-3-isopropylaminopropyloxy)}phenyl)-1-methyläthyl]-2-hydroxy-2-(3-trifluormethylphenyl)äthanamin;
N-[2-(4-{1-(2-Hydroxy-3-dimethylaminopropyloxy)}phenyl)-1-methyläthyl]-2-hydroxy-2-(3-chlorphenyl)äthanamin;
N-[2-(4-{1-2-Hydroxy-3-isopropylaminopropyloxy)}phenyl)1-methyläthyl]-2-hydroxy-2-(3-chlorphenyl) äthanamin.

**Revendications**

1. Composé représenté par la formule (I) ci-après:

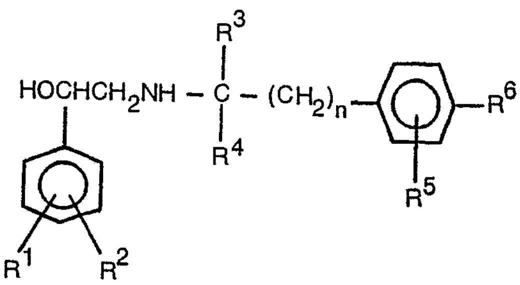

(I)

ou un sel de celui-ci, dans laquelle:

$R^1$ est un atome d'hydrogène, d'halogène ou un groupe trifluorométhyle,

$R^2$ est un atome d'hydrogène ou d'halogène,

$R^3$ est un atome d'hydrogène ou un groupe méthyle,

$R^4$ est un atome d'hydrogène ou un groupe méthyle,

$R^5$est un atome d'hydrogène ou d'halogène,

$R^6$ est 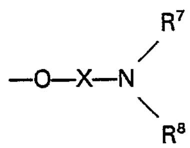

X est un groupe alcoylène en $C_{3-20}$ à chaîne droite ou ramifiée, ayant au moins deux atomes de carbone intercalés entre les atomes d'oxygène et d'azote et ayant un substituant hydroxy sur un atome de carbone qui n'est pas directement relié aux atomes d'oxygène ou d'azote,

$R^7$ est un atome d'hydrogène, un groupe alcoyle en $C_{1-6}$ à chaîne droite ou ramifiée ou benzyle éventuellement substitué,

$R^8$ est un atome d'hydrogène, un groupe alcoyle en $C_{1-6}$ à chaîne droite ou ramifiée ou benzyle éventuellement substitué ou

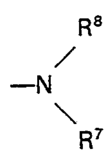

est un groupe hétérocyclique saturé à 5, 6 ou 7 chaînons contenant un total d'un ou deux hétéroatomes, le second hétéroatome, s'il est présent, étant choisi parmi les atomes d'azote, d'oxygène et de soufre, et n est 1 ou 2.

2. Composé suivant la revendication 1, caractérisé en ce que $R^1$ est un atome d'hydrogène, de chlore ou un groupe trifluorométhyle.

3. Composé suivant la revendication 1, caractérisé en ce que $R^6$ est un groupe

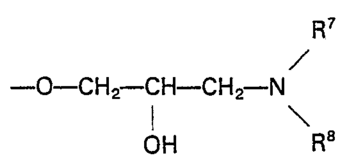

4. Composé suivant la revendication 1, caractérisé en ce que $R^2$ est un atome d'hydrogène.

5. Procédé de production d'un composé de formule (I) suivant la revendication 1, ou d'un sel de celui-ci, caractérisé en ce qu'on réduit un groupe oxo et/ou une double liaison et/ou détache un groupe benzyle d'un composé représenté par la formule (II):

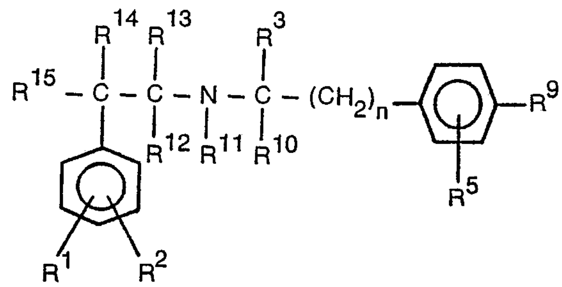

(II)

dans laquelle $R^1$, $R^2$, $R^3$, $R^5$ et n sont tels que définis à propos de la formule (I);

$R^9$ est un groupe $R^6$ tel que défini à propos de la formule (I) ou un groupe réductible en un groupe $R^6$,

$R^{10}$ est un groupe $R^4$ tel que défini à propos de la formule (I) ou il forme une liaison avec $R^{11}$,

$R^{11}$ est un atome d'hydrogène ou un groupe benzyle, ou il forme une liaison avec $R^{10}$ ou $R^{12}$,

$R^{12}$ est un atome d'hydrogène ou il forme une liaison avec $R^{11}$ ou un groupe oxo avec $R^{13}$,

$R^{13}$ est un atome d'hydrogène ou il forme un groupe oxo avec $R^{12}$,

$R^{14}$ est un atome d'hydrogène ou il forme un groupe oxo avec $R^{15}$,

$R^{15}$ est un groupe hydroxy ou il forme un groupe oxo avec $R^{14}$, à condition qu'il n'y ait pas plus d'un groupe oxo représenté par l'un quelconque des $R^{10}$ à $R^{15}$, et éventuellement, on forme ensuite un sel du composé de formule (I) ainsi obtenu, et/ou on convertit le composé de formule (I) ainsi préparé en un autre composé de formule (I).

6. Procédé de production d'un composé de formule (I) tel que défini dans la revendication 1, ou d'un sel de celui-ci, caractérisé en ce qu'on fait réagir un composé de formule (III) ci-après:

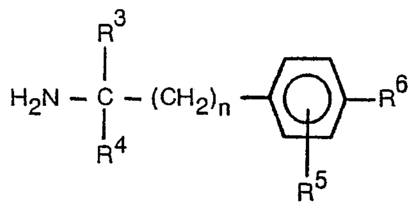

(III)

dans laquelle $R^3$, $R^4$, $R^5$, $R^6$ et n sont tels que définis à propos de la formule (I), avec un composé de formule (IV) ci-après:

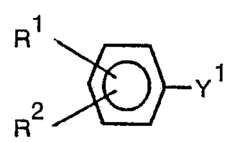

(IV)

dans laquelle $R^1$ et $^2$ sont tels que définis à propos de la formule (I), et $Y^1$ est un groupe capable de réagir avec le groupe amine de la formule (III), en formant ainsi un composé de formule (I), et éventuellement, on forme ensuite un sel du composé de formule (I) ainsi obtenu et/ou on convertit le composé de formule (I) ainsi préparé en un autre composé de formule (I).

7. Procédé de production d'un composé de formule (I) tel que défini dans la revendication 1, ou des sels de celui-ci, caractérisé en ce qu'on fait réagir un composé de formule (V) ci-après:

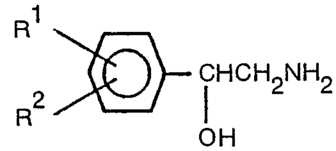

(V)

dans laquelle $R^1$ et $R^2$ sont tels que définis à propos de la formule (I) avec un composé de formule (VI) ci-après:

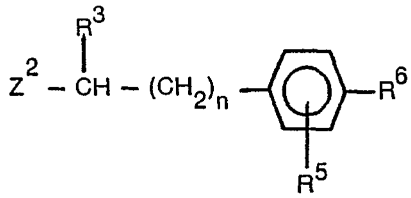

(VI)

dans laquelle $R^3$, $R^5$, $R^6$ et n sont tels que définis à propos de la formule (I) et $Z^2$ est un groupe mobile qui est de préférence un atome d'halogène ou un groupe tosyloxy.

8. Composition pharmaceutique caractérisée en ce qu'elle comprend un composé de formule (I) suivant la revendication 1 et un support acceptable du point de vue pharmaceutique pour celui-ci.

9. Composition suivant la revendication 8, caractérisée en ce qu'elle se trouve sous forme de dose unitaire comprenant de 0,1 à 500 mg du composé de formule (I).

10. Composé de formule (I) utile dans le traitement des hommes et des animaux.

11. Composé suivant la revendication 1, caractérisé en ce qu'il est choisi parmi:

la N-[2-(4-{1-(2-hydroxy-3-isopropylaminopropyloxy)}phényl)-1-méthyléthyl]-2-hydroxy-2-(3-trifluorométhylphényl)éthanamine;

la N-[2-(4-{1-(2-hydroxy-3-diméthylaminopropyloxy)}phényl)-1-méthyléthyl]-2-hydroxy-2-(3-chlorophényl)éthanamine; et

la N-[2-(4-{1-(2-hydroxy-3-isopropylaminopropyloxy)}phényl)-1-méthyléthyl]-2-hydroxy-2-(3-chlorophényl)éthanamine.